# FASCICULE DE BREVET EUROPEEN

(11) **EP 0 781 602 B1**
(45) Date de publication et mention de la délivrance du brevet: **12.09.2001**
(21) Numéro de dépôt: 96402569.6
(22) Date de dépôt: 28.11.1996
(51) Int. Cl.: B01J 21/08, B01J 21/06, C07D 301/12, C07D 301/19, B01J 37/00

(54) **Procédé d'obtention d'un solide à base de silice et de titane et utilisation de ce solide notamment dans l'époxydation des oléfines**
Verfahren zur Herstellung von einem Festkörper auf Basis von Siliziumdioxid und Titan, und seine Anwendung für die Epoxidierung von Olefinen
Process for making a solid based on silica and titanium and use of this solid for the epoxydation of olefins

(30) Priorité: 27.12.1995 FR 9515537
(43) Date de publication de la demande: 02.07.1997
(73) Titulaire: Atofina, 92800 Puteaux (FR)
(72) Inventeur: Jorda, Eric, 69001 Lyon (FR); Teissier, Rémy, 69340 Francheville (FR); Tuel, Alain, 69100 Villeurbanne (FR); Kervennal, Jacques, 69005 Lyon (FR)

(56) Documents cités:
- WO-A-94/23834
- FR-A- 2 295 010
- US-A- 4 367 342

## Description

La présente invention concerne un procédé d'obtention d'un solide à base de silice particulaire comportant du titane et l'utilisation de ce solide, comme catalyseur notamment dans l'époxydation des oléfines.

Les solides à base de silice et de titane sont utilisés comme catalyseurs dans un vaste domaine de réactions chimiques d'oxydation, notamment l'époxydation d'oléfines par le peroxyde d'hydrogène ou les hydroperoxydes organiques, l'ammoximation de cétones et l'hydroxylation du phénol.

Ces catalyseurs ont fait l'objet de nombreuses publications et leur préparation consiste en général à imprégner une silice commerciale de grande surface spécifique par une solution aqueuse de tétrachlorure de titane contenant de l'acide chlorhydrique (US 4 968 842) ou une solution aqueuse de trifluorure de titane contenant de l'acide fluorhydrique (P.J. Kooyman et al. dans "Proceedings 9th International Zeolite Conference" (1993) vol. 1 p. 505-512).

Les catalyseurs à base de silice et de titane peuvent également être préparés en milieu organique anhydre (US 4 021 454).

D'après Paolo ROFFIA et al. (La Chimica & Industria 72, p. 598-603 (1990)), la préparation des catalyseurs à base de silice et de titane par la méthode d'imprégnation conduit essentiellement à des amas de dioxyde de titane. Or, les amas de dioxyde de titane, présentant une absorption caractéristique à 30 500 cm⁻¹ (328 nm) en UV visible est connu pour catalyser la décomposition du peroxyde d'hydrogène utilisé comme agent oxydant dans de nombreuses réactions.

Dans le brevet FR 2 704 159, la demanderesse a réussi à obtenir des catalyseurs solides à base de silice particulaire amorphe, comportant essentiellement des atomes de titane (IV) isolés et montrant en spectrographie UV-visible de réflexion une bande à environ 240 nm.

Ce catalyseur peut être obtenu en imprégnant une silice amorphe par du trifluorure de titane ou du tétrafluorure de titane en milieu organique ou aqueux et éventuellement en calcinant sous air cette silice imprégnée préalablement séchée.

Ainsi l'exemple 9 du brevet FR 2 704 159 décrit la préparation du catalyseur en dissolvant sous agitation 1 g de tétrafluorure de titane dans le 'diglyme à 60° C et en additionnant ensuite 25 g de la silice DEGUSSA FK 310. Après une heure à 60° C sous agitation, le solide est alors filtré puis séché sous pression de 1,33.10² Pa pendant 14 heures. Le solide séché contient 1,9 % poids/poids de fluor.

Pour réduire la teneur en fluor dans ce solide, de manière à préserver l'environnement et éviter la corrosion, un traitement supplémentaire, notamment un lavage par une solution aqueuse basique ou une solution aqueuse de peroxyde d'hydrogène est nécessaire. Ainsi, dans l'exemple 11 de ce brevet, le solide obtenu à l'exemple 9 est remis en suspension dans une solution aqueuse de carbonate d'ammonium dont le pH initial est de 8,5. Après stabilisation jusqu'à pH à environ 7,1 le solide est filtré puis séché pendant 14 heures à 120° C sous 1,33.10² Pa. La teneur en fluor dans le solide traité est réduite à 0,3 % poids/poids.

De même dans l'exemple 25, le traitement par le peroxyde d'hydrogène aqueux suivi d'une filtration et d'un séchage conduit à une réduction de la teneur en fluor de 2,2 à 0,2 % poids/poids.

La demanderesse a maintenant découvert un procédé simplifié d'obtention de solide à base de silice particulaire comportant du titane.

Ce procédé présente l'avantage de comporter une seule étape réactionnelle et d'utiliser l'eau comme solvant. Il présente en outre l'avantage de pouvoir fixer le titane sur la silice particulaire avec un rendement élevé.

La présente invention a donc pour objet un procédé d'obtention de solide à base de silice particulaire comportant du titane caractérisé en ce qu'il comprend la mise en contact d'une suspension contenant une silice particulaire amorphe initiale en milieu aqueux à pH basique, avec du trifluorure de titane (TiF₃) ou du tétrafluorure de titane (TiF₄) en une quantité de 0,1 à 10 % en poids de titane (Ti) par rapport au poids de la silice initiale.

Le pH du milieu réactionnel , avant ou après l'introduction du TiF₃ ou TiF₄, peut être ajusté à l'aide d'une solution basique choisie parmi l'hydroxyde de sodium, l'hydroxyde de potassium et l'hydroxyde d'ammonium. Une solution d'hydroxyde d'ammonium étant particulièrement préférée.

Selon la présente invention, le pH de la suspension peut être compris entre 8,5 et 12,5 et de préférence compris entre 9 et 11.

La température du milieu réactionnel est en général de 10 à environ 40° C, de préférence comprise entre 15 et 30° C.

Avantageusement, le solide à base de silice particulaire et comportant du titane est obtenu à température d'environ 20° C.

La mise en contact de la suspension contenant une silice amorphe initiale en milieu aqueux à pH basique avec du trifluorure de titane ou de tétrafluore de titane peut se faire à pression supérieure ou égale à la pression atmosphérique ou sous pression réduite. On utilise de préférence la pression atmosphérique.

On peut laisser la suspension contenant la silice amorphe initiale en contact avec le trifluorure de titane ou le tetrafluorure de titane pendant une période allant de 15 minutes à 4 heures. Cette mise en contact peut être réalisée notamment, en agitant la suspension. Au delà de 4 heures d'agitation on n'observe pas d'amélioration notable.

Toute silice amorphe à grande surface spécifique peut servir de silice initiale. Avantageusement, cette silice est obtenue par précipitation et sa surface spécifique est comprise entre 200 m²/g et 550 m²/g.

De préférence, la silice initiale a une teneur en fer, nickel, chrome ou tout autre métal de transition, inférieure à 200 p.p.m. ou mieux encore inférieure à 100 p.p.m. En effet, ces métaux peuvent entraîner la décomposition du peroxyde d'hydrogène selon une réaction du type Fenton.

Bien qu'on puisse, après la mise en contact, utiliser la suspension telle quelle on préfère le plus souvent séparer le solide obtenu de la solution aqueuse, notamment par filtration. Le solide ainsi séparé peut être soumis à un séchage. De préférence, le solide séparé et éventuellement séché est soumis à une calcination pratiquée à une température de 200 à 700° C.

Le solide à base de silice particulaire comportant du titane obtenu suivant le procédé de la présente invention contient essentiellement des atomes de titane isolés dans la silice. Ce solide peut être caractérisé par la technique de spectographie U.V avec un rapport r supérieur ou égal à 3.

Le rapport r est le même que celui défini dans le brevet FR 2 704 159, à savoir le rapport de l'intensité d'absorption mesurée à 240 µm sur l'intensité d'absorption mesurée à 330 µm.

Avantageusement, r est égal ou supérieur à 10. De préférence, r est égal ou supérieur à 15.

La teneur en fluor présent dans le solide préparé selon la présente invention est inférieure à 1 % poids/poids, de préférence inférieure à 0,4 % poids/poids.

Les solides obtenus suivant le procédé de la présente invention peuvent être utilisés en tant que catalyseurs pour époxyder une oléfine par du peroxyde d'hydrogène ou un hydroperoxyde organique notamment l'hydroperoxyde de tertiobutyle.

En plus de la description qui précède, l'invention sera mieux comprise à l'aide des exemples qui vont suivre et qui sont donnés à titre illustratif et non limitatif.
- Tétrafluorure de titane ou TiF₄ (Elf - Atochem Ozark Mahoning )
- Trifluorure de titane ou TiF₃ ( Aldrich )

La silice amorphe initiale est une silice DEGUSSA FK 310.

La préparation du solide à base de silice particulaire et comportant du titane se fait généralement en mettant en suspension dans une solution aqueuse à pH basique, la silice particulaire amorphe. Après stabilisation du pH par une solution basique, on introduit du trifluorure ou tetrafluorure de titane préalablement mis en solution dans l'eau désionisée. On laisse le mélange sous agitation à température comprise entre 15 et 30° C pendant une durée variant de 15 minutes à 4 heures, de préférence de 30 minutes à 2 heures. Puis, on filtre et on sèche le solide en étuve à environ 120° C pendant une durée de 6 heures à 15 heures sous pression réduite.

Après séchage, le solide est éventuellement calciné sous air à une température comprise entre 200° C et 700° C pendant quelques heures, de préférence entre 350°C et 500° C pendant 2 à 3 heures.

Le dosage chimique du titane est effectué de manière classique par minéralisation et absorption atomique.

Le dosage chimique du fluor est effectué de manière usuelle par minéralisation, décomplexation et utilisation d'une électrode spécifique commerciale.

La spectroscopie UV-visible de réflexion est effectuée de la même façon que dans le brevet FR 2 704 159.

L'activité catalytique des différents solides est déterminée par une époxydation d'une oléfine réalisée dans des conditions analogues à celles d'une époxydation par le peroxyde d'hydrogène en catalyse homogène. L'oléfine utilisée est le cyclohexène.

La quantité de solide testé est comprise entre 0,5 et 10% en poids par rapport au poids du mélange réactionnel. De préférence cette quantité est de 1,5 à 5% en poids.

Le peroxyde d'hydrogène est introduit sous la forme d'une solution aqueuse à 70% en poids

Le solvant est choisi parmi les solvants classiques utilisés dans l'époxydation par H₂O₂, tels que le diglyme ou le tertiobutanol.

Chaque époxydation a été menée dans un réacteur fermé sur cuve d'eau, afin de déterminer le volume de gaz dégagé et ainsi le taux de décomposition de H₂O₂ en H₂O et O₂ . L'obtention de 10 ml de O₂ gazeux dans les conditions normales de température (20°) et de pression correspond à environ 1 mmole de H₂O₂ décomposé .

### Préparation du solide

### Exemple 1

Dans un réacteur agité, on introduit 50 g d'une solution aqueuse ammoniacale à pH = 10, puis on introduit 5 g de silice Degussa FK 310. On ajuste le pH à 10 par ajout d'une solution d'ammoniaque concentrée jusqu'à stabilisation. On introduit ensuite la solution préalablement préparée contenant 0,2 g de tetrafluorure de titane et 10 g d'eau désionisée.

On laisse sous agitation pendant une heure à température de 20° C, puis on filtre le solide. Le solide est ensuite séché à 120° C sous 1,33.10² Pa pendant 8 heures.

On dose la teneur en fluor et en titane du solide séché. Il contient en poids/poids 1,35 % de Ti et 0,3 % de F. Le rapport r = 25.

### Exemple 2

Cet exemple est réalisé de manière identique à l'exemple 1 mais après le séchage on calcine le solide à 500° C sous air pendant 3 heures.

On dose chimiquement le fluor et le titane :
Ti = 1,45 %
F = 0,35 %
le rapport r = 20

### Exemple 3

Cet exemple est réalisé de manière identique à l'exemple 1 sauf que 0,2 g de tetrafluorure de titane est remplacé par 0,13g de tetrafluorure de titane.

Le solide séché contient :
Ti = 0,9 %
F = 0,2 %
le rapport r = 30

### Exemple 4

Cet exemple est réalisé de manière identique à l'exemple 3 mais après le séchage, on calcine le solide à 500° C sous air pendant 3 heures.

On dose chimiquement le fluor et le titane :
Ti = 1 %
F = 0,2 %
le rapport r = 25

### Exemple 5

Cet exemple est réalisé de manière identique à l' exemple 1 sauf que 0,2 g de tetrafluorure de titane est remplacé par 0,16 g de trifluorure de titane.

Après séchage, le solide est calciné à 500° C sous air.

On dose chimiquement le fluor et le titane :
Ti = 1,4 %
F = 0,2 %

Dans les exemples précédents c'est-à-dire 1 à 5, le taux de fixation du titane (la quantité de titane fixé sur la silice par rapport au titane introduit ) est supérieur à 95 %.

### Exemple 6 (comparatif)

On reproduit l'exemple 14 du brevet FR 2 704 159 qui comporte deux traitements par le carbonate de sodium pour réduire le taux de fluor dans le solide final.

### Epoxydation par H₂O₂

Dans un réacteur agité de 0,5 l thermostaté, muni d'un condenseur à reflux, d'une prise de température, et d'un dispositif d'introduction d'un réactif liquide, on introduit la charge suivante :
- 26 g de diglyme
- 41g de cyclohexène (0,5 mole)
- 2g de catalyseur obtenu dans l'un des exemples 1 à 6 selon le tableau I ci après.

Le condenseur à reflux est relié à une cuve à eau par l'intermédiaire de tuyau et de dispositifs de sécurité anti-retour classiques.

On porte le milieu réactionnel sous agitation et à une température déterminée, en général à 80° C.

On introduit alors en une heure 0,025 mole (0,61 g) de H₂O₂ à 70% en poids dans l'eau en solution dans 10g de diglyme.

Après l'introduction, on laisse réagir à la température déterminée pendant une durée choisie entre une et quatre heures. On observe au niveau de la cuve à eau l'éventuel dégagement de gaz

On laisse refroidir. On vérifie par dosage la quantité de H₂O₂ résiduelle dans le milieu réactionnel (oxydation de Kl en milieu acide, puis dosage de l'iode par du thiosulfate de sodium (selon "G. Chariot - Analyse quantitative minérale p.80 - Masson 1955").

On dose les époxydes par le dosage classique au HCl en milieu éthanolique en présence de chlorure de magnésium (selon "Organic analysis I p. 127 - Interscience 1953").

On analyse le mélange réactionnel par chromatographie gazeuse (chromatographie H.P. 5890, colonne capillaire de diamètre 0,53 mm d'une longueur de 30 m, garnie de carbowax, le gaz vecteur était l'hélium. Le programme : 5 minutes à 70°C puis montée à 12°C/min. jusqu'à 240°C. L'étalonnage est externe). On mesure ainsi la teneur en oxyde de cyclohexène et la teneur en 1,2 - Cyclohexanediol.

Le tableau I montre les résultats obtenus, avec la conversion et les sélectivités par rapport au peroxyde d'hydrogène.

Conversion en % = 100.([H₂O₂]ₒ - [H₂O₂]_{f})/[H₂O₂]ₒ

Sélectivité en époxy en % = 100.([époxy]/([H₂O₂]ₒ-[H₂O₂]_{f})

Sélectivité en diol en % = 100.([diol-1,2]/( [H₂O₂]ₒ-[H₂O₂]_{f})

[H₂O₂]ₒ représente le nombre de moles de peroxyde d'hydrogène introduit dans le milieu réactionnel.
[H₂O₂]_{f} représente le nombre de moles de peroxyde d'hydrogène restant dans le milieu en fin d'opération.
[époxy] représente le nombre de moles d'époxy dans le milieu, en fin d'opération.
[diol - 1,2] représente le nombre de moles de diol - 1,2 dans le milieu, en fin d'opération.

**Tableau 1**

| **Solide** | **Durée** | **Conversion H202** | **Sélectivité epoxyde** | **Sélectivité diol 1,2** |
|---|---|---|---|---|
| ***Exemple 1*** | 1,5 h | 95 % | 70 % | 22 % |
| ***Exemple 2*** | 1,5 h | 95 % | 84 % | 15 % |
| ***Exemple 3*** | 3 h | 95 % | 84 % | 7 % |
| ***Exemple 4*** | 3 h | 91 % | 80 % | 3 % |
| ***Exemple 5*** | 3 h | 92 % | 52 % | 37 % |
| ***Exemple 6*** | 1,5 h | 95 % | 80 % | 15 % |

Dans les exemples 1 à 5, on n' observe pas de dégagement de gaz. Par conséquent la totalité d' eau oxygénée transformée est utilisée pour l' oxydation.

### Epoxydation par l'hydroperoxyde de tertiobutyle.(tBuOOH)

Dans un réacteur identique à celui utilisé ci-dessus pour l'époxydation au peroxyde d'hydrogène, on introduit :
- 2 g du solide obtenu à l'exemple 2, 4 et 6
- 41g (0,5 mole) du cyclohexène
- 40g de cyclohexane.

On chauffe ce mélange hétérogène au reflux qui débute à 75°C. On coule en une heure 0,05 moles d'hydroperoxyde de tertiobutyle dissous dans 20 g de cyclohexane.

Après l'introduction, on laisse réagir à une température voisine de 80° C pendant une durée choisie entre 1 heure et 5 heures pour atteindre une conversion en hydroperoxyde de tertiobutyle égale ou supérieure à 95 %.

Les résultats sont représentés dans le tableau 2.

**Tableau 2**

| **Solide** | **Durée** | **Conversion tBuOOH** | **Sélectivité epoxyde** | **Sélectivité diol 1,2** |
|---|---|---|---|---|
| ***Exemple 2*** | 3 h | 100 % | 90 % | 0 % |
| ***Exemple 4*** | 4 h | 95 % | 93 % | 0 % |
| ***Exemple* 6** | 5 h | 95 % | 50 % | 0 % |

Quel que soit l'agent oxydant utilisé, les solides à base de silice comportant du titane préparés selon l'invention sont très sélectifs dans la réaction d'époxydation.

## Revendications

1. Procédé d'obtention de solide à base de silice particulaire comportant du titane **caractérisé en ce qu'**il comprend la mise en contact d'une suspension contenant une silice particulaire amorphe initiale en milieu aqueux à pH basique avec du trifluorure de titane (TiF₃) ou du tetrafluorure de titane (TiF₄) en une quantité de 0,1 à 10 % en poids de titane (Ti) par rapport au poids de la silice.

2. Procédé suivant la revendication 1 **caractérisé en ce que** le pH est compris entre 8,5 et 12,5.

3. Procédé suivant la revendication 2 **caractérisé en ce que** le pH est compris entre 9 et 11.

4. Procédé suivant l'une des revendications 1 à 3 **caractérisé en ce que** le solide en suspension dans le milieu aqueux est filtré puis séché.

5. Procédé suivant la revendication 4 **caractérisé en ce que** le solide séché est calciné à une température comprise ente 200 et 700°C.

6. Utilisation du solide préparé selon l'une des revendications 1 à 5 en tant que catalyseur.

7. Procédé d'époxydation d'une oléfine par du peroxyde d'hydrogène ou un hydroperoxyde organique, en présence d'un solide à base de silice particulaire comportant du titane **caractérisé en ce que** le solide est préparé selon l'une des revendications 1 à 5.

## Claims

1. Process for obtaining a particulate silica-based solid comprising titanium, **characterized in that** it comprises the placing in contact of a suspension containing an initial amorphous particulate silica in aqueous medium at basic pH with titanium trifluoride (TiF₃) or titanium tetrafluoride (TiF₄) in an amount of from 0.1% to 10% by weight of titanium (Ti) relative to the weight of silica.

2. Process according to Claim 1, **characterized in that** the pH is between 8.5 and 12.5.

3. Process according to Claim 2, **characterized in that** the pH is between 9 and 11.

4. Process according to one of Claims 1 to 3, **characterized in that** the solid suspended in the aqueous medium is filtered off and then dried.

5. Process according to Claim 4, **characterized in that** the dried solid is calcined at a temperature of between 200°C and 700°C.

6. Use of the solid prepared according to one of Claims 1 to 5 as a catalyst.

7. Process for epoxidizing an olefin with hydrogen peroxide or an organic hydroperoxide, in the presence of a particulate silica-based solid comprising titanium, **characterized in that** the solid is prepared according to one of Claims 1 to 5.

## Patentansprüche

1. Verfahren zur Herstellung eines Feststoffs auf der Basis einer partikelförmigen Kieselsäure, der Titan enthält, **dadurch gekennzeichnet, daß** es das Inkontaktbringen einer Suspension, die eine als Ausgangsstoff eingesetzte amorphe, partikelförmige Kieselsäure in wäßrigem Medium mit basischem pH-Wert enthält, mit Titantrifluorid (TiF₃) oder Titantetrafluorid (TiF₄) in einer Menge von 0,1 bis 10 Gew.-% Titan (Ti), bezogen auf das Gewicht der Kieselsäure, umfaßt.

2. Verfahren nach Anspruch 1, **dadurch gekennzeichnet, daß** der pH-Wert im Bereich von 8,5 bis 12,5 liegt.

3. Verfahren nach Anspruch 2, **dadurch gekennzeichnet, daß** der pH-Wert im Bereich von 9 bis 11 liegt.

4. Verfahren nach einem der Ansprüche 1 bis 3, **dadurch gekennzeichnet, daß** der in Suspension in dem wäßrigen Medium enthaltene Feststoff abfiltriert und dann getrocknet wird.

5. Verfahren nach Anspruch 4, **dadurch gekennzeichnet, daß** der getrocknete Feststoff bei einer Temperatur von 200 bis 700 °C calciniert wird.

6. Verwendung des nach einem der Ansprüche 1 bis 5 hergestellten Feststoffs als Katalysator.

7. Verfahren zur Epoxidierung eines Olefins mit Wasserstoffperoxid oder einem organischen Hydroperoxid in Gegenwart eines Feststoffs auf der Basis einer partikelförmigen Kieselsäure, der Titan enthält, **dadurch gekennzeichnet, daß** der Feststoff nach einem der Ansprüche 1 bis 5 hergestellt wird.
